# EUROPEAN PATENT SPECIFICATION

(11) **EP 3 845 132 B1**
(45) Date of publication and mention of the grant of the patent: **15.04.2026**
(21) Application number: 19854516.2
(22) Date of filing: 26.06.2019
(51) Int. Cl.: A61B 8/14, A61B 8/00, A61B 8/08

(54) **ULTRASONIC DIAGNOSTIC DEVICE AND ULTRASONIC DIAGNOSTIC DEVICE CONTROL METHOD**
DIAGNOSTISCHE ULTRASCHALLVORRICHTUNG UND STEUERUNGSVERFAHREN FÜR DIAGNOSTISCHE ULTRASCHALLVORRICHTUNG
DISPOSITIF DE DIAGNOSTIC À ONDES ULTRASONORES ET PROCÉDÉ DE COMMANDE DE DISPOSITIF DE DIAGNOSTIC À ONDES ULTRASONORES

(30) Priority: 29.08.2018 JP 2018160282
(43) Date of publication of application: 07.07.2021
(73) Proprietor: FUJIFILM Corporation, Tokyo 106-8620 (JP)
(72) Inventor: MATSUMOTO Tsuyoshi, Ashigarakami-gun, Kanagawa 258-8538 (JP)
(74) Representative: Dehns Germany Partnerschaft mbB
(86) International application number: PCT/JP2019/025420
(87) International publication number: WO 2020/044758

(56) References cited:
- WO-A1-2018/035392
- JP-A- 2007 532 152
- JP-A- 2013 017 870
- JP-A- 2015 027 400
- US-A1- 2004 221 853
- US-A1- 2017 189 634
- TSUNG JAMES W ET AL: "Dynamic anatomic relationship of the esophagus and trachea on sonography: implications for endotracheal tube confirmation in children", JOURNAL OF ULTRASOUND IN MEDICINE, 1 September 2012 (2012-09-01), England, pages 1365 - 1370, XP055841801, Retrieved from the Internet <URL:https://onlinelibrary.wiley.com/doi/epdf/10.7863/jum.2012.31.9.1365> [retrieved on 20210916], DOI: 10.7863/jum.2012.31.9.1365
- HOFFMANN B. ET AL: "Bedside Ultrasound of the Neck Confirms Endotracheal Tube Position in Emergency Intubations", vol. 35, no. 05, 1 January 2013 (2013-01-01), DE, pages 451 - 458, XP055841554, ISSN: 0172-4614, Retrieved from the Internet <URL:https://www.thieme-connect.de/products/ejournals/pdf/10.1055/s-0034-1366014.pdf> DOI: 10.1055/s-0034-1366014
- YOU-TEN KONG ERIC ET AL: "Point-of-care ultrasound (POCUS) of the upper airway", CANADIAN JOURNAL OF ANAESTHESIA / JOURNAL CANADIEN D'ANESTHESIE, CANADIAN ANAESTHETISTS SOCIETY, TORONTO, CA, vol. 65, no. 4, 18 January 2018 (2018-01-18), pages 473 - 484, XP036676765, ISSN: 0832-610X, [retrieved on 20180118], DOI: 10.1007/S12630-018-1064-8
- HAO-CHANG CHOU ET AL: "Tracheal rapid ultrasound exam (T.R.U.E.) for confirming endotracheal tube placement during emergency intubation", RESUSCITATION, ELSEVIER, IE, vol. 82, no. 10, 14 May 2011 (2011-05-14), pages 1279 - 1284, XP028296763, ISSN: 0300-9572, [retrieved on 20110601], DOI: 10.1016/J.RESUSCITATION.2011.05.016

## Description

### BACKGROUND OF THE INVENTION

### 1. Field of the Invention

The present invention relates to an ultrasound diagnostic apparatus and a method of controlling an ultrasound diagnostic apparatus, and particularly, to an ultrasound diagnostic apparatus and a method of controlling an ultrasound diagnostic apparatus that are used for observing a trachea and an esophagus of a subject.

### 2. Description of the Related Art

In the related art, tracheal intubation in which the tracheal tube is inserted into the trachea of the subject has been known as a measure for securing an airway of the subject. In a state where tracheal intubation is performed, for example, oxygen is supplied from the tracheal tube to the trachea of the subject to secure the airway of the subject. In addition, in a case where tracheal intubation is performed, so-called esophageal intubation may be performed in which the tracheal tube is inserted into the esophagus instead of the trachea of the subject. Since esophageal intubation causes choking of the subject, the presence or absence of esophageal intubation is necessarily quickly confirmed during tracheal intubation, and in a case where esophageal intubation is detected, the tracheal tube is necessarily quickly removed from the esophagus.

In recent years, as a method of quickly confirming the presence or absence of the esophageal intubation, a method of directly confirming the presence or absence of the esophageal intubation by observing an esophagus of the subject by using the ultrasound diagnostic apparatus has attracted attention. Generally, the ultrasound diagnostic apparatus is an apparatus that obtains an image of the inside of the subject, and comprises an ultrasound probe comprising an oscillator array in which a plurality of elements are arranged. In a state where the ultrasound probe is brought into contact with a body surface of the subject, an ultrasound beam is transmitted from the oscillator array toward the inside of the subject, ultrasound echoes from the subject are received by the oscillator array, and element data are acquired. Moreover, the ultrasound diagnostic apparatus processes the obtained element data electrically, and generates an ultrasound image for a relevant part of the subject.

JP2007-532152A discloses an ultrasound diagnostic apparatus that generates a so-called Doppler image based on the ultrasound echo from the subject. In JP2007-532152A, a vibration mechanism is connected to the tracheal tube, and the ultrasound probe is brought into contact with the throat of the subject in a state where the tracheal tube is vibrated in the subject. In this state, the Doppler image is generated, and the tracheal tube is visualized in the generated Doppler image. Tsung James W Et al. Journal of ultrasound in medicine 2012-09-01 pages 1365-1370, XP055841801 discloses sonographic visualization of an empty esophagus. Hoffman B. Et al. Ultraschall in der medizin - European journal of ultrasound, vol. 34, no. 05, 2013-01-01, pages 451-458, XP055841554 discloses bedside ultrasound of the neck in emergency intubations. You-ten Kong Eric Et al. Canadian Journal of Anesthesia, vol. 65, no. 4, 2018-01-18, pages 473-484, XP036676765 discloses an overview of point-of-care ultrasound of the upper airway. US 2017/189634 discloses a system, method and device for airway assessment an endotracheal intubation.

Further, document WO2018035392 discloses systems and methods for identification of anatomical structures using ultrasound imaging. This document mentions that said systems and methods can be used to distinguish placement of an intubation tube in the trachea or esophagus.

### SUMMARY OF THE INVENTION

In the subject, the esophagus is often positioned on the left side of the trachea as viewed from the subject, but depending on the subject, it may be positioned on the right side of the trachea. In the ultrasound diagnostic apparatus disclosed in JP2007-532152A, the vibration of the tracheal tube is merely visualized, and it is difficult to determine whether the tracheal tube is inserted into the trachea or the esophagus. In particular, in a case where the esophagus is positioned on the right side of the trachea, there is a risk of overlooking the esophageal intubation.

In a case where a less skilled user confirms the presence or absence of the esophageal intubation, there is a risk of overlooking the esophageal intubation due to impatience during an emergency.

The present invention has been made in order to solve such related-art problems, and an object thereof is to provide an ultrasound diagnostic apparatus and a method of controlling an ultrasound diagnostic apparatus that can confirm the presence or absence of the esophageal intubation regardless of the skill of the user.

According to an aspect of the present invention, there is provided an ultrasound diagnostic apparatus as claimed in claim 1.

It is preferable that the operation guiding unit guide the user to position the ultrasound probe in an order of a position suitable for observing the trachea, and the position suitable for observing the esophagus.

In this case, it is preferable that the operation guiding unit guide the user to position the ultrasound probe on a left side of the trachea as viewed from the subject for observation of the esophagus, and in a case where the esophagus is not detected by the observation target detecting unit at a position on the left side of the trachea, guide the user to position the ultrasound probe on a right side of the trachea as viewed from the subject.

Further, in a case where the esophagus is not detected by the observation target detecting unit on the right side of the trachea as viewed from the subject, the operation guiding unit may guide the user to position the ultrasound probe on the left side of the trachea and the right side of the trachea while compressing a throat of the subject.

The observation target detecting unit may detect the esophagus by performing the image analysis using a first template pattern representing only the esophagus, and a second template pattern representing the esophagus into which the tracheal tube is inserted with respect to the ultrasound image acquired by the image acquisition unit, and in a case where the esophagus corresponding to the second template pattern is detected by the observation target detecting unit, the esophagus state determination unit may determine that the tracheal tube is inserted into the esophagus.

Alternatively, in a case where a ratio of a size of the esophagus to a size of the trachea detected by the observation target detecting unit is larger than a predetermined value, the esophagus state determination unit may determine that the tracheal tube is inserted into the esophagus.

It is preferable that the esophagus state determination unit notify the user of a determination result representing whether the tracheal tube is inserted into the esophagus.

The ultrasound diagnostic apparatus may further comprise a display unit, in which the operation guiding unit causes the display unit to display operation guidance on the ultrasound probe for the user, and the esophagus state determination unit causes the display unit to display the determination result representing whether the tracheal tube is inserted into the esophagus.

According to a second aspect, there is provided a computer implemented method of controlling the ultrasound diagnostic apparatus as claimed in claim 9.

According to the present invention, the ultrasound diagnostic apparatus comprises an observation target detecting unit that detects the trachea and the esophagus included in the ultrasound image by performing image analysis with respect to the ultrasound image acquired by the image acquisition unit, an operation guiding unit that guides a user to operate the ultrasound probe such that the ultrasound probe is positioned at a position suitable for observing the esophagus based on a detection result by the observation target detecting unit, and an esophagus state determination unit that determines whether a tracheal tube is inserted into the esophagus detected by the observation target detecting unit, and thus it is possible to confirm the presence or absence the esophageal intubation regardless of the skill of the user.

### BRIEF DESCRIPTION OF THE DRAWINGS

Fig. 1 is a block diagram showing a configuration of an ultrasound diagnostic apparatus according to Embodiment 1 of the present invention.
Fig. 2 is a block diagram showing an internal configuration of a receiving unit according to Embodiment 1 of the present invention.
Fig. 3 is a block diagram showing an internal configuration of an image generation unit according to Embodiment 1 of the present invention.
Fig. 4 is a diagram schematically showing a trachea and an esophagus in a case where esophageal intubation is not performed.
Fig. 5 is a diagram schematically showing a trachea and an esophagus in a case where esophageal intubation is performed.
Fig. 6 is a diagram schematically showing a cross section of the throat of a subject.
Fig. 7 is a flowchart showing an operation of the ultrasound diagnostic apparatus according to Embodiment 1 of the present invention.
Fig. 8 is a diagram showing an example of operation guidance in Embodiment 1 of the present invention.
Fig. 9 is a diagram showing another example of operation guidance in Embodiment 1 of the present invention.
Fig. 10 is a flowchart showing an operation of the ultrasound diagnostic apparatus according to Embodiment 2 of the present invention.

### DESCRIPTION OF THE PREFERRED EMBODIMENTS

Hereinafter, embodiments of the present invention will be described based on the accompanying drawings.

### Embodiment 1

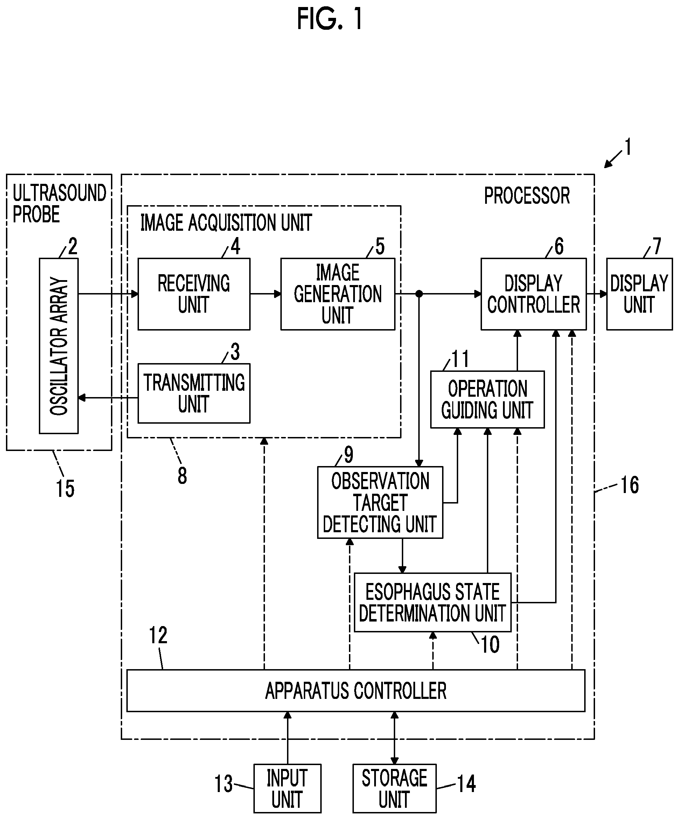

A configuration of an ultrasound diagnostic apparatus 1 according to Embodiment 1 of the present invention is shown in Fig. 1. As shown in Fig. 1, the ultrasound diagnostic apparatus 1 comprises an oscillator array 2, and a transmitting unit 3 and a receiving unit 4 are connected to the oscillator array 2. An image generation unit 5, a display controller 6, and a display unit 7 are sequentially connected to the receiving unit 4. An image acquisition unit 8 is configured by the transmitting unit 3, the receiving unit 4, and the image generation unit 5. An observation target detecting unit 9 is connected to the image generation unit 5, and an esophagus state determination unit 10 is connected to the observation target detecting unit 9. The esophagus state determination unit 10 is connected to the display controller 6. An operation guiding unit 11 is connected to the observation target detecting unit 9 and the esophagus state determination unit 10, and the display controller 6 is connected to the operation guiding unit 11.

The apparatus controller 12 is connected to the display controller 6, the image acquisition unit 8, the observation target detecting unit 9, the esophagus state determination unit 10, the operation guiding unit 11, and the input unit 13 and the storage unit 14 are connected to the apparatus controller 12. The apparatus controller 12 and the storage unit 14 are connected to each other so as to be capable of transferring information bidirectionally.

The oscillator array 2 is included in the ultrasound probe 15, and a processor 16 is configured by the display controller 6, the image acquisition unit 8, the observation target detecting unit 9, the esophagus state determination unit 10, the operation guiding unit 11, and the apparatus controller 12.

The oscillator array 2 of the ultrasound probe 15 shown in Fig. 1 has a plurality of oscillators arranged in one dimension or two dimensions. These oscillators transmit ultrasound waves in accordance with drive signals supplied from the transmitting unit 3, respectively, and receive ultrasound echoes from the subject to output the received signals. The respective oscillators are configured by, for example, forming electrodes to both ends of piezoelectric material made of a piezoelectric ceramic represented by lead zirconate titanate (PZT), a polymeric piezoelectric element represented by polyvinylidene difluoride (PVDF), and a piezoelectric single crystal represented by a lead magnesium niobate-lead titanate solid solution (PMN-PT).

The transmitting unit 3 of the image acquisition unit 8 includes, for example, a plurality of pulse generators, and adjust the amounts of delay of the respective drive signals to supply the adjusted drive signals to the plurality of oscillators such that the ultrasound waves transmitted from the plurality of oscillators of the oscillator array 2 form an ultrasound beam, based on a transmission delay pattern selected in accordance with the control signals from the apparatus controller 12. In this way, in a case where a pulsed or consecutive wave-like voltage is applied to electrodes of the plurality of oscillators of the oscillator array 2, the piezoelectric material expands and contracts, a pulsed or consecutive wave-like ultrasound wave is generated from the respective oscillators, and the ultrasound beam is formed from a synthetic wave of the ultrasound waves.

The transmitted ultrasound beam is reflected in, for example, targets such as a part of the subject and is propagated toward the oscillator array 2 of the ultrasound probe 15. The ultrasound echoes propagated toward the oscillator array 2 in this way are received by the respective oscillators that constitute the oscillator array 2. In this case, the respective oscillators that constitute the oscillator array 2 expand and contract by receiving the propagated ultrasound echoes, to generate electrical signals, and output the electrical signals to the receiving unit 4.

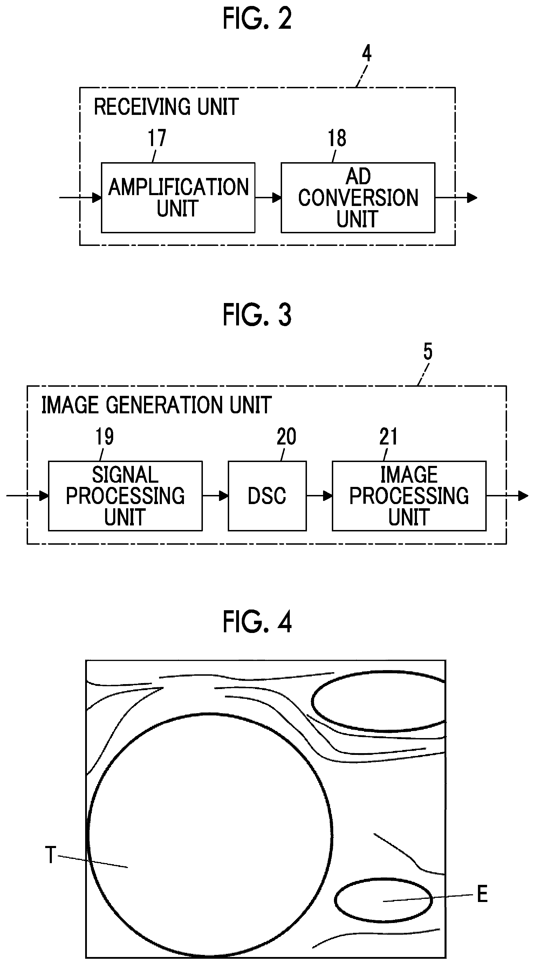

The receiving unit 4 of the image acquisition unit 8 processes the received signals output from the oscillator array 2 in accordance with the control signals from the apparatus controller 12. As shown in Fig. 2, the receiving unit 4 has a configuration that an amplification unit 17 and an analog-digital (AD) conversion unit 18 are serially connected. The amplification unit 17 amplifies the received signals input from the respective oscillators that constitute the oscillator array 2, and transmits the amplified received signals to the AD conversion unit 18. The AD conversion unit 18 converts the received signals transmitted from the amplification unit 17 into digitalized data, and sends the data to the image generation unit 5 of the image acquisition unit 8.

As shown in Fig. 3, the image generation unit 5 of the image acquisition unit 8 has a configuration that a signal processing unit 19, a digital scan converter (DSC) 20, and an image processing unit 21 are serially connected. The signal processing unit 19 performs reception focus processing that addition (phasing addition) is performed by giving each delay to each data of the received signals, based on a reception delay pattern selected in accordance with the control signals from the apparatus controller 12. With the reception focus processing, sound ray signals in which focal points of the ultrasound echoes are narrowed to one scan line are generated. Additionally, the signal processing unit 19 subjects the generated sound ray signals to the correction of damping resulting from a propagation distance depending on the depth at a position where the ultrasound waves are reflected, and then performs envelope detection processing to generate B-mode image signals indicating a tissue in the subject. The B-mode image signals generated in this way are output to the DSC 20.

The DSC 20 of the image generation unit 5 raster-converts the B-mode image signals into image data based on a scan mode of normal television signals to generate the ultrasound image. The image processing unit 21 of the image generation unit 5 subjects the ultrasound image obtained in the DSC 20 to various kinds of required image processing such as brightness correction, grayscale correction, sharpness correction, and color correction, and then outputs an ultrasound image to the display controller 6, and the observation target detecting unit 9.

The observation target detecting unit 9 of the processor 16 performs image analysis with respect to the ultrasound image acquired by the image acquisition unit 8, and detects the trachea and the esophagus which are to be observed. More specifically, the observation target detecting unit 9 detects the trachea and the esophagus by recognizing the cross sections of the trachea and the esophagus included in the ultrasound image. The cross section of the trachea is a cross section in a case of cutting the trachea across the central axis of the trachea, and the cross section of the esophagus is a cross section in a case of cutting the esophagus across the central axis of the esophagus. For example, the observation target detecting unit 9 can recognize the cross section of the trachea or the esophagus by storing typical pattern data such as a template image as a template in advance, calculating the similarity to the pattern data while searching the image with the template, and assuming the trachea and the esophagus are present in the position where the similarity is equal to or larger than a threshold value and is maximum.

A machine learning method such as a so-called Adaboost, and a method disclosed in Csurka et al.: Visual Categorization with Bags of Keypoints, Proc. of ECCV Workshop on Statistical Learning in Computer Vision, pp.59-74 (2004), a general image recognition method using deep learning by a deep neural network of multi layers described in Krizhevsky et al.: Image Net Classification with Deep Convolutional Neural Networks, Advances in Neural Information Processing Systems 25, and pp.1106-1114 (2012), or the like can be used for the calculation of the similarity in addition to the simple template matching. In the machine learning method, for example, a detection target such as the trachea and the esophagus is detected using a so-called feature amount vector, and in a general image method using deep learning, for example, a detection target is detected using a weight in a so-called artificial neuron.

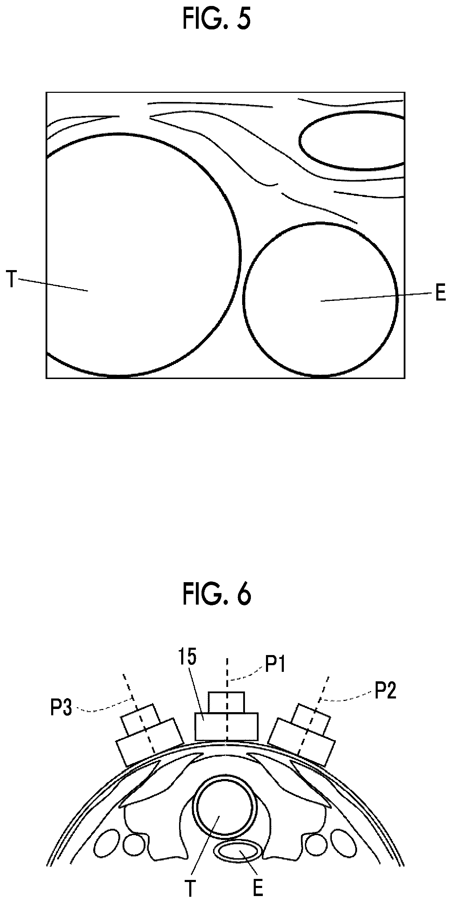

The esophagus state determination unit 10 of the processor 16 determines whether the tracheal tube is inserted into the esophagus detected by the observation target detecting unit 9, and notifies the user of the determination result. Fig. 4 schematically shows the ultrasound image including the cross sections of the esophagus E and the trachea T into which the tracheal tube is not inserted, and Fig. 5 schematically shows the ultrasound image including the cross sections of the esophagus E and the trachea T into which the tracheal tube is inserted. Figs. 4 and 5 are the schematic diagrams of the ultrasound image generated in a state where the ultrasound probe 15 is brought into contact with the throat of the subject from the front, the upper side of the figure corresponds to the front surface side of the subject, and the lower side of the figure corresponds to the rear surface side of the subject. In Figs. 4 and 5, the front side of the paper surface corresponds to the leg side of the subject, and the back side of the paper surface corresponds to the head side of the subject.

In a case where the tracheal tube is not inserted into the esophagus, as shown in the schematic diagram of Fig. 4, the size of the esophagus E is sufficiently small as compared with the size of the trachea T, but in a case where the tracheal tube is inserted into the esophagus, as shown in the schematic diagram of Fig. 5, the esophagus E is expanded by the tracheal tube, and the size of the esophagus E is larger than the size in the state shown in Fig. 4. Therefore, in a case where the observation target detecting unit 9 detects the esophagus by performing the image analysis using a first template pattern representing a normal esophagus in which the tracheal tube is not inserted, and a second template pattern representing the esophagus into which the tracheal tube is inserted, the esophagus state determination unit 10 can determine that the tracheal tube is inserted into the esophagus in a case where the esophagus corresponding to the second template pattern is detected by the observation target detecting unit 9.

The operation guiding unit 11 of the processor 16 guides the user to operate the ultrasound probe 15 based on the detection result by the observation target detecting unit 9 such that the ultrasound probe 15 is positioned in a position suitable for detecting the esophagus. Fig. 6 shows a cross-sectional view of the throat of the subject across the trachea T and the esophagus E of the subject. Fig. 6 shows a state in which the esophagus E exists on the left side of the trachea T as viewed from the subject, the front side of the paper surface corresponds to the leg side of the subject, and the back side of the paper surface corresponds to the head side of the subject. The left side of the trachea T as viewed from the subject is, for example, the left hand side of the subject with reference to the trachea T. That is, in a case where the user views the subject from the front with the subject's head up and legs down, the left side of the trachea T as viewed from the subject is the right side of the trachea T as viewed from the user. The right side of the trachea T as viewed from the subject is, for example, the right hand side of the subject with reference to the trachea T.

Further, the esophagus E is hidden behind the trachea T, and statistically, the position of the esophagus E with respect to the trachea T is positioned on the left side of the trachea T as viewed from the subject in about 80% of the subjects, and is positioned on the right side of the trachea T as viewed from the subject about 20% of the subjects. As described above, the position of the esophagus E is often positioned on the left side of the trachea T as viewed from the subject, for example, the operation guiding unit 11 first guides the ultrasound probe 15 to be positioned in a predetermined position P1 in the front surface as viewed from the subject in order to obtain a cross section of the trachea T, and then, guides the ultrasound probe 15 to be positioned in a predetermined position P2 on the left side of the position P1 as viewed from the subject in order to obtain a cross section of the esophagus E. In a case where the cross section of the esophagus E cannot be obtained at the position P2, the operation guiding unit 11 can guide the user to position the ultrasound probe 15 in a predetermined position P3 on the right side of the position P1 as viewed from the subject.

In this case, the operation guiding unit 11 can guide the user to operate the ultrasound probe 15 by, for example, displaying the text and the image indicating the guidance for the user on the display unit 7. Additionally, although not shown, for example, a voice generation unit that generates voice can be provided in the ultrasound diagnostic apparatus 1, and the operation guiding unit 11 can also guide the user by the voice via the voice generation unit.

The apparatus controller 12 of the processor 16 performs control of the respective units of the ultrasound diagnostic apparatus 1 based on the programs that are stored in advance in the storage unit 14 and the like and the operation of the user via the input unit 13.

Under the control of the apparatus controller 12, the display controller 6 of the processor 16 displays the ultrasound image generated by the image generation unit 5 of the image acquisition unit 8 on the display unit 7.

The display unit 7 of the ultrasound diagnostic apparatus 1 displays the ultrasound image under the control of the display controller 6, and includes display devices such as a liquid crystal display (LCD), and an organic electroluminescence (EL) display.

The input unit 13 of the ultrasound diagnostic apparatus 1 is a device for the user to perform input operation, and can be configured to comprise a keyboard, a mouse, a trackball, a touchpad, a touch panel, and the like.

The storage unit 14 stores operating programs and the like of the ultrasound diagnostic apparatus 1, and as the storage unit 14, a recording medium such as a hard disc drive (HDD), a solid state drive (SSD), a flexible disc (FD), a magneto-optical (MO) disc, a magnetic tape (MT), a random access memory (RAM), a compact disc (CD), a digital versatile disc (DVD), a secure digital (SD) card, a universal serial bus (USB) memory, or the like, or a server can be used.

In addition, the processor 16 having the display controller 6, the image acquisition unit 8, the observation target detecting unit 9, the esophagus state determination unit 10, the operation guiding unit 11, and the apparatus controller 12 is configured from a central processing unit (CPU) and control programs for making the CPU perform various kinds of processing. However, the processor 16 may be configured from a field programmable gate array (FPGA), a digital signal processor (DSP), an application specific integrated circuit (ASIC), or other integrated circuits (ICs), or may be configured by combining them.

Also, the display controller 6, the image acquisition unit 8, the observation target detecting unit 9, the esophagus state determination unit 10, the operation guiding unit 11, and the apparatus controller 12 of the processor 16 can be partially or entirely integrated into one CPU.

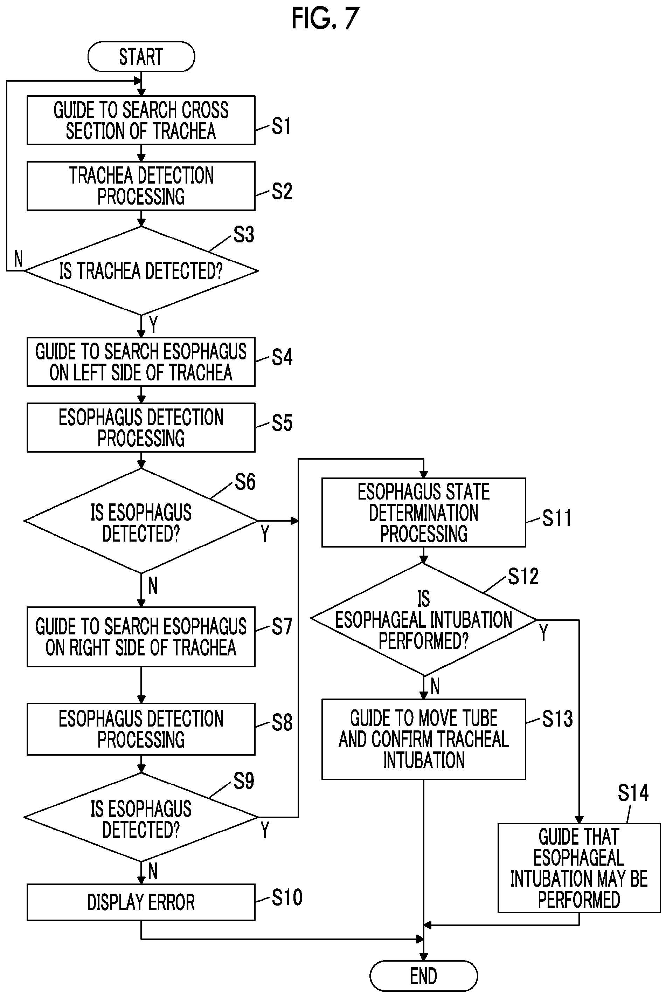

Next, the operation of the ultrasound diagnostic apparatus 1 in Embodiment 1 will be described in detail using a flowchart shown in Fig. 7. In the following, it is assumed that the ultrasound diagnostic apparatus 1 performs ultrasound diagnosis on the throat of the subject in a state where the tracheal tube is inserted in the throat of the subject.

First, in step S1, the operation guiding unit 11 guides the user to search the position of the ultrasound probe 15 suitable for detecting the trachea T. Although not shown, in this case, the operation guiding unit 11 can display, for example, text indicating searching the cross section of the trachea T on the display unit 7. In this way, in a case where the operation guiding unit 11 provides the guidance to the user, the user operates the ultrasound probe 15 to position the ultrasound probe 15 on the position P1 shown in Fig. 6 as a position suitable for detecting the trachea T.

In this way, the ultrasound waves are transmitted to and received from the oscillator array 2 of the ultrasound probe 15 positioned at the position P1 with respect to the throat of the subject, and the ultrasound image is acquired by the image acquisition unit 8.

Next, in step S2, the observation target detecting unit 9 performs processing of detecting the cross section of the trachea T by performing the image analysis with respect to the ultrasound image acquired by the image acquisition unit 8.

In following step S3, the operation guiding unit 11 determines whether the cross section of the trachea T is detected in step S2 by receiving the information indicating the result of the detection processing performed in step S2 from the observation target detecting unit 9. In step S3, in a case where the determination is made that the cross section of the trachea T is not detected by the detection processing of step S2, the process returns to step S1, and the operation guiding unit 11 provides the guidance for searching the position of the ultrasound probe 15 in order to acquire the ultrasound image including the cross section of the trachea T. In a case where the user moves the ultrasound probe 15 in accordance with the guidance, in step S2, the observation target detecting unit 9 performs processing of detecting the cross section of the trachea T by performing the image analysis with respect to the acquired ultrasound image. In following step S3, the operation guiding unit 11 newly determines whether the cross section of the trachea T is detected in step S2.

In this way, the processing of step S1 to step S3 are repeated until the cross section of the trachea T is detected in step S2.

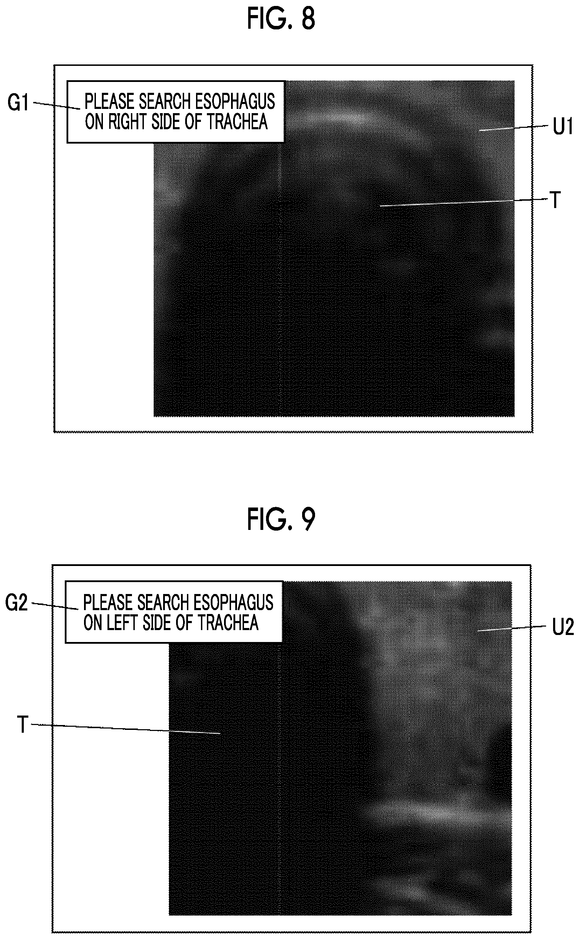

In step S3, in a case where the determination is made that the cross section of the trachea T is detected by the detection processing of step S2, the process proceeds to step S4. In step S4, the operation guiding unit 11 guides the user to move the ultrasound probe 15 to the left side of the trachea T as viewed from the subject as a position suitable for detecting the esophagus E, and to search the esophagus E. The position on the left side of the trachea T is a position, such as the position P2 shown in Fig. 6, suitable for observing the esophagus E positioned behind the trachea T from the left side of the trachea T as viewed from the subject in a case where the esophagus E is positioned on the left side of the trachea T as viewed from the subject. In this case, as shown in Fig. 8, the operation guiding unit 11 can guide the user to operate the ultrasound probe 15 by, for example, displaying a guide panel G1 including the text indicating the guidance for the user on the display unit 7. In a case where the user views the subject from the front, the left side of the trachea T as viewed from the subject is the right side of the trachea T as viewed from the user, and thus in the example shown in Fig. 8, the guide panel G1 including a text indicating "please search esophagus on right side of trachea" is superimposed on the ultrasound image U1 including trachea T. In a case where the operation of the ultrasound probe 15 is guided to the user, the ultrasound probe 15 is moved by the user to the left side of the trachea T as viewed from the subject, for example, to the position P2 shown in Fig. 6. Further, in a state where the ultrasound probe 15 is positioned on the left side of the trachea T as viewed from the subject, the ultrasound image is acquired by the image acquisition unit 8.

In following step S5, the observation target detecting unit 9 performs processing of detecting the cross section of the esophagus E by performing the image analysis with respect to the ultrasound image acquired by the image acquisition unit 8. In this case, as shown in Figs. 4 and 5, the observation target detecting unit 9 can detect the esophagus E by performing the image analysis using a first template pattern representing the esophagus E in which the tracheal tube is not inserted into the esophagus E, and a second template pattern representing the esophagus E in which the tracheal tube is inserted into the esophagus E. Hereinafter, for the sake of explanation, it is assumed that the observation target detecting unit 9 performs the detection processing of the esophagus E using the first template pattern and the second template pattern.

In step S6, the operation guiding unit 11 determines whether the cross section of the esophagus E is detected in step S5 by receiving the information indicating the result of the detection processing performed in step S5 from the observation target detecting unit 9. In step S6, in a case where the determination is made that the cross section of the esophagus E is not detected by the detection processing of step S5, the process proceeds to step S7.

In step S7, the operation guiding unit 11 guides the user to move the ultrasound probe 15 to the right side of the trachea T as viewed from the subject as a position suitable for detecting the esophagus E, and to search the esophagus E. The position on the right side of the trachea T is a position, such as the position P3 shown in Fig. 6, suitable for observing the esophagus E positioned behind the trachea T in a case where the esophagus E is positioned on the right side of the trachea T as viewed from the subject. In this case, as shown in Fig. 9, the operation guiding unit 11 can guide the user to operate the ultrasound probe 15 by, for example, displaying a guide panel G2 including the text indicating the guidance for the user on the display unit 7. In a case where the user views the subject from the front, the right side of the trachea T as viewed from the subject is the left side of the trachea T as viewed from the user, and thus in the example shown in Fig. 9, the guide panel G2 including a text indicating "please search esophagus on left side of trachea" is superimposed on the ultrasound image U2 including trachea T. In a case where the operation of the ultrasound probe 15 is guided to the user, the ultrasound probe 15 is moved by the user to the right side of the trachea T as viewed from the subject, for example, to the position P3 shown in Fig. 6. Further, in a state where the ultrasound probe 15 is positioned on the right side of the trachea T as viewed from the subject, the ultrasound image is acquired by the image acquisition unit 8.

Generally, in the subject, the esophagus E is often positioned on the left side of the trachea T as viewed from the subject, but it may be positioned on the right side of the trachea T. In the ultrasound diagnostic apparatus 1 of Embodiment 1, in a case where the esophagus E cannot be detected on the left side of the trachea T as viewed from the subject, the operation guiding unit 11 guides the user to search the esophagus E on the right side of the trachea T as viewed from the subject, and thus even a less skilled user can more reliably search the esophagus E.

In following step S8, the observation target detecting unit 9 performs processing of detecting the cross section of the esophagus E by performing the image analysis with respect to the ultrasound image acquired by the image acquisition unit 8, as in step S5.

In step S9, the operation guiding unit 11 determines whether the cross section of the esophagus E is detected in step S8 by receiving the information indicating the result of the detection processing performed in step S8 from the observation target detecting unit 9. In step S9, in a case where the determination is made that the cross section of the esophagus E is not detected by the detection processing of step S8, the process proceeds to step S10, the fact that an error occurs is displayed on the display unit 7, and the operation of the ultrasound diagnostic apparatus 1 ends.

In a case where the determination is made that the cross section of the esophagus E is detected by the detection processing of step S5 in step S6, or in a case where the determination is made the cross section of the esophagus E is detected by the detection processing of step S8 in step S9, the process proceeds to step S11.

In step S11, the esophagus state determination unit 10 determines whether the tracheal tube is inserted into the esophagus E detected in step S8. In this case, for example, the esophagus state determination unit 10 determines that the tracheal tube is not inserted into the esophagus E in a case where the observation target detecting unit 9 detects the cross section of the esophagus E using the first template pattern, and determines that the tracheal tube is inserted into the esophagus E in a case where the second template pattern is used for detection. As a result, even a less skilled user can easily confirm the presence or absence of esophageal intubation.

In following step S12, the esophagus state determination unit 10 determines the presence or absence of so-called esophageal intubation in which the tracheal tube is inserted into the esophagus E detected in step S5 or step S8. In a case where the determination is made that the esophageal intubation is not performed in step S12, the process proceeds to step S13.

In step S13, the esophagus state determination unit 10 guides the user to move the tracheal tube inserted into the throat of the subject and confirm whether the tracheal tube is inserted into the trachea, as a determination result indicating that the esophageal intubation is not performed. In this way, in a case where the guidance of step S13 is completed, the operation of the ultrasound diagnostic apparatus 1 ends.

In a case where the determination is made that the esophageal intubation is performed in step S12, the process proceeds to step S14. In step S14, the esophagus state determination unit 10 guides the user that there is a suspicion of esophageal intubation, as a determination result indicating that esophageal intubation is performed. In this way, in a case where the guidance of step S14 is completed, the operation of the ultrasound diagnostic apparatus 1 ends.

As described above, in the ultrasound diagnostic apparatus 1 according to Embodiment 1, the operation guiding unit 11 guides the user to search the esophagus E on the left side and the right side of the trachea T as viewed from the subject, the esophagus state determination unit 10 determines the presence or absence of esophageal intubation in a case where the esophagus E is detected by the observation target detecting unit 9, and the esophagus state determination unit 10 provides the guidance to the user as a result of the determination result, and thus it is possible to confirm the presence or absence of the esophageal intubation regardless of the skill of the user.

In Embodiment 1, the esophagus state determination unit 10 determines whether the tracheal tube is inserted into the detected esophagus E based on whether which of the first template pattern or the second template pattern is used for detection of the esophagus E by the observation target detecting unit 9, but a method of determining whether the tracheal tube is inserted into the esophagus E is not particularly limited.

For example, the observation target detecting unit 9 can detect the cross section of the esophagus E regardless of the presence or absence of the esophageal intubation by performing the image processing such as edge detection on the ultrasound image instead of detecting the cross section of the esophagus E using the first template pattern and the second template pattern. In this case, the esophagus state determination unit 10 can distinguish the presence or absence of the esophageal intubation using a so-called neural network with respect to the esophagus E detected by the observation target detecting unit 9.

As shown in Figs. 4 and 5, since the cross section of the esophagus E with the esophageal intubation is larger than the cross section of the esophagus E without the esophageal intubation, for example, it is possible that the esophagus state determination unit 10 calculates the area of the esophagus E included in the ultrasound image, determines that the esophageal intubation is performed in a case where the calculated area is larger than the typical area of the esophagus E, and determines that the esophageal intubation is not performed in a case where the calculated area is smaller than the typical area. Also, it is possible that the esophagus state determination unit 10 calculates the inner diameter of the cross section of the esophagus E, determines that the esophageal intubation is performed in a case where the calculated inner diameter is larger than the typical inner diameter of the esophagus E, and determines that the esophageal intubation is not performed in a case where the calculated inner diameter is smaller than the typical inner diameter.

The typical area and inner diameter of the esophagus E can be stored in the esophagus state determination unit 10 in advance as a predetermined threshold value.

For example, the esophagus state determination unit 10 can determine that the esophageal intubation is performed in a case where a ratio of the size of the esophagus E to the size of the trachea T included in the ultrasound image is larger than a predetermined threshold value, and determine that the esophageal intubation is not performed in a case where the ratio is smaller than a predetermined threshold value. As the size of the trachea T and the size of the esophagus E, for example, the area of the cross section of the trachea T and the area of the cross section of the esophagus E included in the ultrasound image, the inner diameter of the cross section of the trachea T and the inner diameter of the cross section of the esophagus E included in the ultrasound image, or the like can be used.

In the example shown in Fig. 8, a guide panel G1 including a text indicating "please search esophagus on right side of trachea" is displayed on the display unit 7 as the guidance for positioning the ultrasound probe 15 on the left side of the trachea T as viewed from the subject, and in the example shown in Fig. 9, a guide panel G2 including a text indicating "please search esophagus on left side of trachea" is displayed on the display unit 7 as the guidance for positioning the ultrasound probe 15 on the right side of the trachea T as viewed from the subject, but the present invention is not particularly limited thereto. For example, in a case where an inspection protocol such as the user's standing position and the posture of the subject in a case of inspecting the throat of the subject is defined, the content of the guidance provided by the operation guiding unit 11 is appropriately changed in accordance with the defined protocol. For example, depending on the defined protocol, a text "please search esophagus on left side of trachea" may be displayed on the display unit 7 as the guidance for positioning the ultrasound probe 15 on the left side of the trachea T as viewed from the subject. For example, the content of guidance provided by the operation guiding unit 11 can be changed by the user via the input unit 13.

### Embodiment 2

In Embodiment 1, in a case where the esophagus E cannot be detected on the left side of the trachea T as viewed from the subject, the operation guiding unit 11 guides the user to position the ultrasound probe 15 on the right side of the trachea T as viewed from the subject, but depending on the subject, the esophagus E may be positioned behind the trachea T, and it may be difficult to detect the esophagus E. In such a case, for example, a method of observing the throat of the subject while shifting the position of the esophagus E by compressing the throat of the subject is effective.

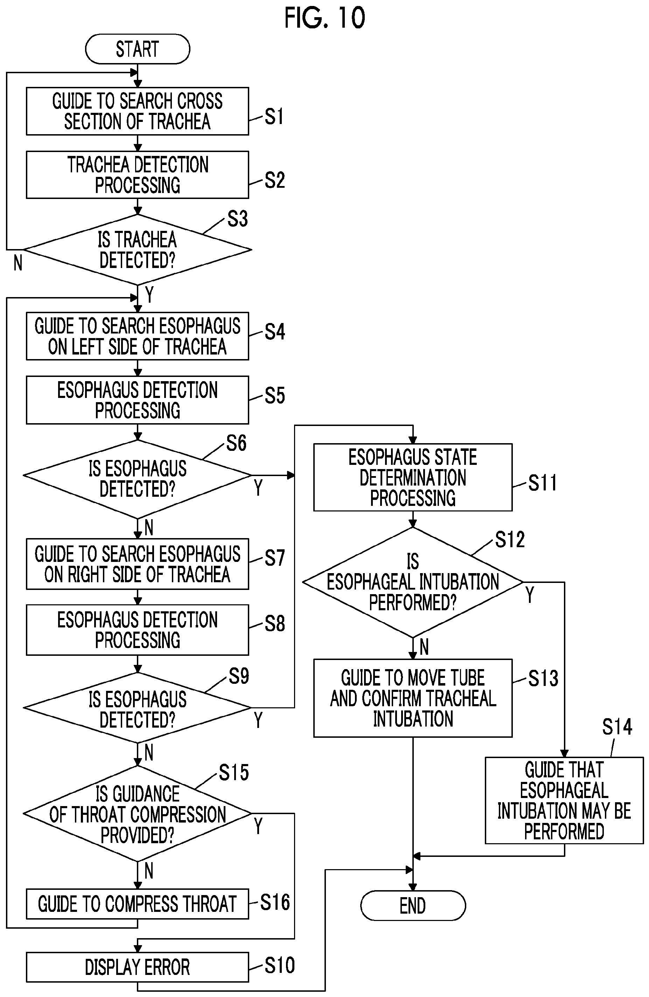

Fig. 10 shows a flowchart showing an operation of the ultrasound diagnostic apparatus 1 according to Embodiment 2. The flowchart is a flowchart in which step S15 and step S16 are added after step S9 of the flowchart shown in Fig. 7.

First, in step S1, the operation guiding unit 11 guides the user to position the ultrasound probe 15 on the position in which the ultrasound image including the cross section of the trachea T is acquired. The user positions the ultrasound probe 15 on, for example, the position P1 shown in Fig. 6 in accordance with the guidance in step S1. Further, the image acquisition unit 8 acquires the ultrasound image in a state where the ultrasound probe 15 is positioned at the position P1.

In step S2, the observation target detecting unit 9 performs processing of detecting the cross section of the trachea T included in the ultrasound image.

In following step S3, the operation guiding unit 11 determines whether the cross section of the trachea T is detected in step S2. In step S3, in a case where the determination is made that the cross section of the trachea T is not detected by the detection processing of step S2, the process returns to step S1, and the processing of step S1 to step S3 are performed again. In step S3, in a case where the determination is made that the cross section of the trachea T is detected by the detection processing of step S2, the process proceeds to step S4.

In step S4, the operation guiding unit 11 guides the user to move the ultrasound probe 15 to the left side of the trachea T as viewed from the subject and search the esophagus E. The user positions the ultrasound probe 15 on the left side of the trachea T as viewed from the subject, for example, the position P2 shown in Fig. 6 in accordance with the guidance in step S4. Further, the image acquisition unit 8 acquires the ultrasound image in a state where the ultrasound probe 15 is positioned at the position P2.

In step S5, the observation target detecting unit 9 performs processing of detecting the cross section of the esophagus E included ultrasound image acquired by the image acquisition unit 8.

In following step S6, the operation guiding unit 11 determines whether the cross section of the esophagus E is detected in step S5. In step S6, in a case where the determination is made that the cross section of the esophagus E is not detected by the detection processing of step S5, the process proceeds to step S7.

In step S7, the operation guiding unit 11 guides the user to position the ultrasound probe 15 on the right side of the trachea T as viewed from the subject and search the esophagus E. The user positions the ultrasound probe 15 on the right side of the trachea T as viewed from the subject, for example, the position P3 shown in Fig. 6 in accordance with the guidance in step S7. Further, the image acquisition unit 8 acquires the ultrasound image in a state where the ultrasound probe 15 is positioned at the position P3.

In step S8, the observation target detecting unit 9 performs processing of detecting the cross section of the esophagus E included ultrasound image acquired by the image acquisition unit 8.

In following step S9, the operation guiding unit 11 determines whether the cross section of the esophagus E is detected in step S8. In step S9, in a case where the determination is made that the cross section of the esophagus E is not detected by the detection processing of step S8, the process proceeds to step S15.

In step S15, the operation guiding unit 11 determines whether the guidance for compressing the throat of the subject has already been provided. Since in step S1 to step S9, the guidance for compressing the throat of the subject is not provided, first, the process proceeds to step S16.

In step S16, the operation guiding unit 11 guides the user to compress the throat of the subject. Although not shown, in this case, the operation guiding unit 11 can provide the guidance to the user by displaying the guide panel including the text indicating compressing of the throat of the subject on the display unit 7.

In this way, in a case where the guidance of step S16 is provided, the process returns to step S4. In step S4, the operation guiding unit 11 guides the user to position the ultrasound probe 15 on the left side of the trachea T as viewed from the subject and search the esophagus E. The user positions the ultrasound probe 15 on the left side of the trachea T as viewed from the subject, for example, the position P2 shown in Fig. 6 while compressing the throat of the subject. In this case, the image acquisition unit 8 acquires the ultrasound image in a state where the throat of the subject is compressed, the position of the trachea T is displaced, and the ultrasound probe 15 is positioned at the position P2.

In following step S5, the observation target detecting unit 9 performs processing of detecting the esophagus E included in the ultrasound image.

In step S6, the operation guiding unit 11 determines whether the esophagus E is detected in step S5. In step S6, in a case where the determination is made that the esophagus E is not detected by the detection processing of step S5, the process proceeds to step S7.

In step S7, the operation guiding unit 11 guides the user to position the ultrasound probe 15 on the right side of the trachea T as viewed from the subject and search the esophagus E. The user positions the ultrasound probe 15 on the right side of the trachea T as viewed from the subject, for example, the position P3 shown in Fig. 6 while compressing the throat of the subject. In this case, the image acquisition unit 8 acquires the ultrasound image in a state where the throat of the subject is compressed, the position of the trachea T is displaced, and the ultrasound probe 15 is positioned at the position P3.

In following step S8, the observation target detecting unit 9 performs processing of detecting the esophagus E included in the ultrasound image.

In step S9, the operation guiding unit 11 determines whether the esophagus E is detected in step S8. In step S9, in a case where the determination is made that the esophagus E is not detected by the detection processing of step S8, the process proceeds to step S15.

In step S15, the determination is made on whether the guidance for compressing the throat of the subject has already been provided by the operation guiding unit 11, but in step S16, the guidance for compressing the throat of the subject has already been provided, and thus the process proceeds to step S10. In step S10, the operation guiding unit 11 causes the display unit 7 to display that an error occurs. In this way, in a case where the processing of step S10 is completed, the operation of the ultrasound diagnostic apparatus 1 according to Embodiment 2 ends.

In a case where the determination is made that the esophagus E is detected by the detection processing of step S5 in step S6, or in a case where the esophagus E is detected by the detection processing of step S8 in step S9, the process proceeds to step S11.

In step S11, the esophagus state determination unit 10 determines whether the tracheal tube is inserted into the esophagus E detected in step S5 or step S8.

In following step S12, the esophagus state determination unit 10 determines the presence or absence of the esophageal intubation based on the determination result of step S11. In a case where the determination is made that the esophageal intubation is not performed in step S12, the process proceeds to step S13. In step S13, the esophagus state determination unit 10 guides the user to move the tracheal tube and confirm whether the tracheal tube is inserted into the trachea T. In this way, in a case where the guidance of step S13 is completed, the operation of the ultrasound diagnostic apparatus 1 according to Embodiment 2 ends.

In a case where the determination is made that the esophageal intubation is performed in step S12, the process proceeds to step S14. In step S14, the esophagus state determination unit 10 guides the user that there is a suspicion of esophageal intubation. In this way, in a case where the guidance of step S14 is completed, the operation of the ultrasound diagnostic apparatus 1 according to Embodiment 2 ends.

As described above, in the ultrasound diagnostic apparatus 1 according to Embodiment 2, in a case where the esophagus cannot be detected on the right and left sides of the trachea T as viewed from the subject, the operation guiding unit 11 provides the guidance for compressing the throat of the subject, and provides the guidance for newly searching the esophagus on the right and left sides of the trachea T as viewed from the subject, and thus it is possible to more reliably confirm the presence or absence the esophageal intubation.

### Explanation of References

1: ultrasound diagnostic apparatus
2: oscillator array
3: transmitting unit
4: receiving unit
5: image generation unit
6: display controller
7: display unit
8: image acquisition unit
9: observation target detecting unit
10: esophagus state determination unit
11: operation guiding unit
12: apparatus controller
13: input unit
14: storage unit
15: ultrasound probe
16: processor
17: amplification unit
18: AD conversion unit
19: signal processing unit
20: DSC
21: image processing unit
E: esophagus
G1, G2: guide panel
P1, P2, P3: position
T: trachea
U1, U2: ultrasound image

## Claims

1. An ultrasound diagnostic apparatus (1) adapted to observe a trachea and an esophagus of a subject, the apparatus comprising:
an ultrasound probe (15);
an image acquisition unit (8) that transmits an ultrasound beam toward the subject from the ultrasound probe (15) to acquire an ultrasound image when the ultrasound probe (15) is brought into contact with a throat of the subject from the front; the apparatus **characterised by**:
an observation target detecting unit (9) that detects the trachea and the esophagus included in the ultrasound image by performing image analysis with respect to the ultrasound image acquired by the image acquisition unit (8);
an operation guiding unit (11) that guides a user to operate the ultrasound probe (15) such that the ultrasound probe (15) is positioned at a position suitable for observing the esophagus based on a detection result by the observation target detecting unit (9); and
an esophagus state determination unit (10) that determines whether a tracheal tube is inserted into the esophagus detected by the observation target detecting unit (9).

2. The ultrasound diagnostic apparatus according to claim 1,
wherein the operation guiding unit (11) guides the user to position the ultrasound probe (15) in an order of a position suitable for observing the trachea, and the position suitable for observing the esophagus.

3. The ultrasound diagnostic apparatus according to claim 2,
wherein the operation guiding unit (11) guides the user to position the ultrasound probe (15) on a left side of the trachea as viewed from the subject for observation of the esophagus, and in a case where the esophagus is not detected by the observation target detecting unit (9) at a position on the left side of the trachea, guides the user to position the ultrasound probe on a right side of the trachea as viewed from the subject.

4. The ultrasound diagnostic apparatus according to claim 3,
wherein in a case where the esophagus is not detected by the observation target detecting unit (9) on the right side of the trachea as viewed from the subject, the operation guiding unit (11) guides the user to position the ultrasound probe on the left side of the trachea and the right side of the trachea while compressing a throat of the subject.

5. The ultrasound diagnostic apparatus according to any one of claims 1 to 4,
wherein the observation target detecting unit (9) detects the esophagus by performing the image analysis using a first template pattern representing only the esophagus, and a second template pattern representing the esophagus into which the tracheal tube is inserted with respect to the ultrasound image acquired by the image acquisition unit (8), and
in a case where the esophagus corresponding to the second template pattern is detected by the observation target detecting unit (9), the esophagus state determination unit (10) determines that the tracheal tube is inserted into the esophagus.

6. The ultrasound diagnostic apparatus according to any one of claims 1 to 4,
wherein in a case where a ratio of a size of the esophagus to a size of the trachea detected by the observation target detecting unit (9) is larger than a predetermined value, the esophagus state determination unit determines (10) that the tracheal tube is inserted into the esophagus.

7. The ultrasound diagnostic apparatus according to any one of claims 1 to 6,
wherein the esophagus state determination unit (10) notifies the user of a determination result representing whether the tracheal tube is inserted into the esophagus.

8. The ultrasound diagnostic apparatus according to claim 7, further comprising a display unit (7),
wherein the operation guiding unit (11) causes the display unit (7) to display operation guidance on the ultrasound probe (15) for the user, and
the esophagus state determination unit (10) causes the display unit (7) to display the determination result representing whether the tracheal tube is inserted into the esophagus.

9. A computer implemented method of controlling the ultrasound diagnostic apparatus according to any one of the claims 1-8, the method comprising:
transmitting an ultrasound beam toward a throat of the subject from the ultrasound probe (15) to acquire an ultrasound image when the ultrasound probe (15) is brought into contact with the throat of the subject from the front;
guiding a user to operate the ultrasound probe (15) such that the ultrasound probe (15) is positioned at a position suitable for observing the trachea of the subject, and detecting the trachea included in the ultrasound image by performing image analysis with respect to the acquired ultrasound image;
guiding the user to operate the ultrasound probe (15) such that the ultrasound probe (15) is positioned at a position suitable for observing the esophagus of the subject, and detecting the esophagus included in the ultrasound image by performing the image analysis with respect to the acquired ultrasound image; and
determining whether a tracheal tube is inserted into the esophagus based on the detected esophagus.

## Patentansprüche

1. Ultraschalldiagnosevorrichtung (1), die so angepasst ist, dass sie eine Luftröhre und eine Speiseröhre einer Untersuchungsperson beobachtet, wobei die Vorrichtung umfasst:
eine Ultraschallsonde (15);
eine Bilderfassungseinheit (8), die einen Ultraschallstrahl von der Ultraschallsonde (15) zu der Untersuchungsperson hin transmittiert, um ein Ultraschallbild zu erfassen, wenn die Ultraschallsonde (15) mit einem Hals der Untersuchungsperson von vorne in Kontakt gebracht wird; wobei die Vorrichtung **gekennzeichnet ist durch**:
eine Beobachtungsziel-Detektionseinheit (9), die die Luftröhre und die Speiseröhre, die in dem Ultraschallbild enthalten sind, detektiert, indem sie Bildanalyse in Bezug auf das von der Bilderfassungseinheit (8) erfasste Ultraschallbild durchführt;
eine Bedienungsleiteinheit (11), die einen Benutzer so anleitet, dass er die Ultraschallsonde (15) so bedient, dass die Ultraschallsonde (15) auf der Grundlage eines Detektionsergebnisses **durch** die Beobachtungsziel-Detektionseinheit (9) an einer Position positioniert wird, die zum Beobachten der Speiseröhre geeignet ist; und
eine Speiseröhrenzustands-Bestimmungseinheit (10), die bestimmt, ob ein Trachealtubus in die von der Beobachtungsziel-Detektionseinheit (9) detektierte Speiseröhre eingeführt ist.

2. Ultraschalldiagnosevorrichtung nach Anspruch 1,
wobei die Bedienungsleiteinheit (11) den Benutzer so anleitet, dass er die Ultraschallsonde (15) in einer Reihenfolge einer Position, die zum Beobachten der Luftröhre geeignet ist, und der Position, die zum Beobachten der Speiseröhre geeignet ist, positioniert.

3. Ultraschalldiagnosevorrichtung nach Anspruch 2,
wobei die Bedienungsleiteinheit (11) den Benutzer so anleitet, dass er die Ultraschallsonde (15) auf einer linken Seite der Luftröhre bei Betrachtung von der Untersuchungsperson aus zum Beobachten der Speiseröhre positioniert, und in einem Fall, in dem die Speiseröhre von der Beobachtungsziel-Detektionseinheit (9) an einer Position auf der linken Seite der Luftröhre nicht detektiert wird, den Benutzer so anleitet, dass er die Ultraschallsonde auf einer rechten Seite der Luftröhre bei Betrachtung von der Untersuchungsperson aus positioniert.

4. Ultraschalldiagnosevorrichtung nach Anspruch 3,
wobei in einem Fall, in dem die Speiseröhre von der Beobachtungsziel-Detektionseinheit (9) auf der rechten Seite der Luftröhre bei Betrachtung von der Untersuchungsperson aus nicht detektiert wird, die Bedienungsleiteinheit (11) den Benutzer so anleitet, dass er die Ultraschallsonde auf der linken Seite der Luftröhre und der rechten Seite der Luftröhre positioniert, während er einen Hals der Untersuchungsperson komprimiert.

5. Ultraschalldiagnosevorrichtung nach einem der Ansprüche 1 bis 4,
wobei die Beobachtungsziel-Detektionseinheit (9) die Speiseröhre detektiert, indem sie die Bildanalyse unter Verwendung eines ersten Vorlagenmusters, das nur die Speiseröhre darstellt, und eines zweiten Vorlagenmusters, das die Speiseröhre darstellt, in die der Trachealtubus eingeführt ist, in Bezug auf das von der Bilderfassungseinheit (8) erfasste Ultraschallbild durchführt, und
in einem Fall, in dem die Speiseröhre, die dem zweiten Vorlagenmuster entspricht, von der Beobachtungsziel-Detektionseinheit (9) detektiert wird, die Speiseröhrenzustands-Bestimmungseinheit (10) bestimmt, dass der Trachealtubus in die Speiseröhre eingeführt ist.

6. Ultraschalldiagnosevorrichtung nach einem der Ansprüche 1 bis 4,
wobei in einem Fall, in dem ein Verhältnis einer Größe der Speiseröhre zu einer Größe der Luftröhre, die von der Beobachtungsziel-Detektionseinheit (9) detektiert wird, größer als ein vorbestimmter Wert ist, die Speiseröhrenzustands-Bestimmungseinheit (10) bestimmt, dass der Trachealtubus in die Speiseröhre eingeführt ist.

7. Ultraschalldiagnosevorrichtung nach einem der Ansprüche 1 bis 6,
wobei die Speiseröhrenzustands-Bestimmungseinheit (10) den Benutzer über ein Bestimmungsergebnis benachrichtigt, das darstellt, ob der Trachealtubus in die Speiseröhre eingeführt ist.

8. Ultraschalldiagnosevorrichtung nach Anspruch 7, ferner umfassend eine Anzeigeeinheit (7),
wobei die Bedienungsleiteinheit (11) die Anzeigeeinheit (7) veranlasst, Bedienungsanleitung für die Ultraschallsonde (15) für den Benutzer anzuzeigen, und die Speiseröhrenzustands-Bestimmungseinheit (10) die Anzeigeeinheit (7) veranlasst, das Bestimmungsergebnis anzuzeigen, das darstellt, ob der Trachealtubus in die Speiseröhre eingeführt ist.

9. Computerimplementiertes Verfahren des Steuerns der Ultraschalldiagnosevorrichtung nach einem der Ansprüche 1 bis 8, wobei das Verfahren umfasst:
Transmittieren eines Ultraschallstrahls zu einem Hals der Untersuchungsperson hin von der Ultraschallsonde (15), um ein Ultraschallbild zu erfassen, wenn die Ultraschallsonde (15) von vorne in Kontakt mit dem Hals der Untersuchungsperson gebracht wird;
Anleiten eines Benutzers, die Ultraschallsonde (15) so zu bedienen, dass die Ultraschallsonde (15) an einer Position positioniert wird, die zum Beobachten der Luftröhre der Untersuchungsperson geeignet ist, und Detektieren der Luftröhre, die in dem Ultraschallbild enthalten ist, durch Durchführen von Bildanalyse in Bezug auf das erfasste Ultraschallbild;
Anleiten des Benutzers, die Ultraschallsonde (15) so zu bedienen, dass die Ultraschallsonde (15) an einer Position positioniert wird, die zum Beobachten der Speiseröhre der Untersuchungsperson geeignet ist, und Detektieren der Speiseröhre, die in dem Ultraschallbild enthalten ist, durch Durchführen der Bildanalyse in Bezug auf das erfasste Ultraschallbild; und
Bestimmen, ob ein Trachealtubus in die Speiseröhre eingeführt ist, auf der Grundlage der detektierten Speiseröhre.

## Revendications

1. Appareil de diagnostic par ultrasons (1) adapté pour observer une trachée et un œsophage d'un sujet, l'appareil comprenant :
une sonde ultrasonore (15) ;
une unité d'acquisition d'images (8) qui transmet un faisceau ultrasonore vers le sujet depuis la sonde ultrasonore (15) pour acquérir une image ultrasonore lorsque la sonde ultrasonore (15) est mise en contact avec une gorge du sujet depuis l'avant ; l'appareil étant **caractérisé par** :
une unité de détection de cible d'observation (9) qui détecte la trachée et l'oesophage inclus dans l'image ultrasonore en effectuant une analyse d'image par rapport à l'image ultrasonore acquise par l'unité d'acquisition d'images (8) ;
une unité de guidage d'opération (11) qui guide un utilisateur pour opérer la sonde ultrasonore (15) de sorte que la sonde ultrasonore (15) soit positionnée à une position appropriée pour observer l'oesophage sur la base d'un résultat de détection par l'unité de détection de cible d'observation (9) ; et
une unité de détermination d'état d'oesophage (10) qui détermine si un tube trachéal est inséré dans l'oesophage détecté par l'unité de détection de cible d'observation (9).

2. Appareil de diagnostic par ultrasons selon la revendication 1,
dans lequel l'unité de guidage d'opération (11) guide l'utilisateur pour positionner la sonde ultrasonore (15) dans un ordre d'une position appropriée pour observer la trachée, et la position appropriée pour observer l'oesophage.

3. Appareil de diagnostic par ultrasons selon la revendication 2,
dans lequel l'unité de guidage d'opération (11) guide l'utilisateur pour positionner la sonde ultrasonore (15) sur un côté gauche de la trachée vue du sujet pour l'observation de l'oesophage, et dans un cas où l'oesophage n'est pas détecté par l'unité de détection de cible d'observation (9) à une position sur le côté gauche de la trachée, guide l'utilisateur pour positionner la sonde ultrasonore sur un côté droit de la trachée vue du sujet.

4. Appareil de diagnostic par ultrasons selon la revendication 3,
dans lequel dans un cas où l'oesophage n'est pas détecté par l'unité de détection de cible d'observation (9) sur le côté droit de la trachée vue du sujet, l'unité de guidage d'opération (11) guide l'utilisateur pour positionner la sonde ultrasonore sur le côté gauche de la trachée et le côté droit de la trachée tout en comprimant une gorge du sujet.

5. Appareil de diagnostic par ultrasons selon l'une quelconque des revendications 1 à 4,
dans lequel l'unité de détection de cible d'observation (9) détecte l'oesophage en effectuant l'analyse d'image en utilisant un premier motif modèle représentant uniquement l'oesophage, et un deuxième motif modèle représentant l'oesophage dans lequel le tube trachéal est inséré par rapport à l'image ultrasonore acquise par l'unité d'acquisition d'images (8), et
dans un cas où l'oesophage correspondant au deuxième motif modèle est détecté par l'unité de détection de cible d'observation (9), l'unité de détermination d'état d'oesophage (10) détermine que le tube trachéal est inséré dans l'oesophage.

6. Appareil de diagnostic par ultrasons selon l'une quelconque des revendications 1 à 4,
dans lequel dans un cas où un rapport entre une taille de l'oesophage et une taille de la trachée détectée par l'unité de détection de cible d'observation (9) est supérieur à une valeur prédéterminée, l'unité de détermination d'état d'oesophage (10) détermine que le tube trachéal est inséré dans l'oesophage.

7. Appareil de diagnostic par ultrasons selon l'une quelconque des revendications 1 à 6,
dans lequel l'unité de détermination d'état d'oesophage (10) notifie à l'utilisateur un résultat de détermination représentant si le tube trachéal est inséré dans l'oesophage.

8. Appareil de diagnostic par ultrasons selon la revendication 7, comprenant en outre une unité d'affichage (7),
dans lequel l'unité de guidage d'opération (11) amène l'unité d'affichage (7) à afficher un guidage d'opération sur la sonde ultrasonore (15) pour l'utilisateur, et
l'unité de détermination d'état d'oesophage (10) amène l'unité d'affichage (7) à afficher le résultat de détermination représentant si le tube trachéal est inséré dans l'oesophage.

9. Procédé mis en œuvre par ordinateur pour contrôler l'appareil de diagnostic par ultrasons selon l'une quelconque des revendications 1 à 8, le procédé comprenant :
transmettre un faisceau ultrasonore vers une gorge du sujet depuis la sonde ultrasonore (15) pour acquérir une image ultrasonore lorsque la sonde ultrasonore (15) est mis en contact avec la gorge du sujet depuis l'avant ;
guider un utilisateur pour opérer la sonde ultrasonore (15) de sorte que la sonde ultrasonore (15) soit positionnée à une position appropriée pour observer la trachée du sujet, et détecter la trachée incluse dans l'image ultrasonore en effectuant une analyse d'image par rapport à l'image ultrasonore acquise ;
guider l'utilisateur pour opérer la sonde ultrasonore (15) de sorte que la sonde ultrasonore (15) soit positionnée à une position appropriée pour observer l'oesophage du sujet, et détecter l'oesophage inclus dans l'image ultrasonore en effectuant l'analyse d'image par rapport à l'image ultrasonore acquise ; et
déterminer si un tube trachéal est inséré dans l'oesophage sur la base de l'oesophage détecté.
